# EUROPEAN PATENT SPECIFICATION

(11) **EP 4 308 282 B1**
(45) Date of publication and mention of the grant of the patent: **27.11.2024**
(21) Application number: 22706336.9
(22) Date of filing: 24.02.2022
(51) Int. Cl.: B01J 8/04

(54) **REACTOR SYSTEM FOR MIXING OPERATION AT PARTIAL LOAD**
REAKTORSYSTEM FÜR MISCHBETRIEB BEI TEILLAST
SYSTÈME DE RÉACTEUR POUR MÉLANGER UNE OPÉRATION À UNE CHARGE PARTIELLE

(30) Priority: 16.03.2021 EP 21162870
(43) Date of publication of application: 24.01.2024
(73) Proprietor: CASALE SA, 6900 Lugano (CH)
(72) Inventor: RIZZI, Maurizio, 22079 Villa Guardia (IT)
(74) Representative: M. Zardi & Co S.A.
(86) International application number: PCT/EP2022/054698
(87) International publication number: WO 2022/194513

(56) References cited:
- WO-A1-2013/097958
- US-A- 4 372 920
- US-A- 5 648 051
- US-A1- 2012 305 446
- US-B2- 7 275 565

## Description

### Field of application

The present invention concerns the field of reactor systems for industrial applications such as synthesis of ammonia and methanol. In particular, the present invention concerns a reactor system for improved mixing operation at partial load.

### Prior art

Industrial synthesis reactors are generally conceived to run at full capacity or close to maximum capacity because the plant's economic profitability is expected to drop at reduced capacity.

In more details, at partial load the production rate of chemical converters is reduced and the yield of the chemical reaction may be lower than the expected because mass and heat transfer limitations may become important for example as a consequence of a not adequate mixing between the reactants in the catalytic beds.

A good mixing established inside the chemical converters is essential to guarantee a uniform temperature and composition profile. In the absence of good mixing, no or little reaction will take place. Furthermore, a uniform temperature and composition profile guarantees that the converters work as close as possible to the optimized profile exploiting at best the catalyst volumes.

In addition, a uniform temperature and composition profile prevents the formation of potential by-products, which can negatively affect the process yield and might create purification issues in downstream process units. Lastly, since most of the converters are operated via automatic control loops, it is essential to have reliable leading temperature signals to avoid loss of efficiency in the converter or even unwanted shutdown. Unfortunately, at reduced capacity or low turn down, due to fluid-dynamic reasons, the mixing devices arranged in the converter lose a lot of their effectiveness.

Typically, a suitable mixing between two or more gas reactants in a catalytic converter is achieved by imposing an adequate flow velocity of the reagents fed to the converter. When the operating condition of the converter switches from full to partial load, the flow velocity of reagents provided to the converter may drop under a threshold value where no satisfactory mixing condition can be established so that mass and heat transport limitations may arise thus limiting the maximum conversion achievable, the catalyst selectiveness or even the converter reliability.

Unfortunately, it is not always possible to operate the converters at full capacity. Typically, reactors that may be forced to operate under partial load conditions are the ammonia and the methanol converters when one of the reagents (e.g. hydrogen) is produced using apparatus powered by renewal energy sources. For example, hydrogen can be produced in a water electrolyzer powered by solar or wind energy. Such plants that exploit renewal energy sources to synthesize one of the reagents are known as green plants.

The green plants are of great interest because of the low operational cost and low pollution; for example, they do not produce CO₂ contrary to the conventional coal-based or natural gas-based plants. However, renewable sources like solar or wind are intrinsically subject to fluctuations, e.g. solar energy is not available during night time. Therefore, load fluctuations occurring during the production cycle must be expected and dealt with.

Unsatisfactory mixing conditions may be established not only in the catalytic beds of the converter but as well in the interbed cooling section of the reactor usually interposed between two subsequent catalytic beds. Typically, in the converter's interbed cooling section, the effluent of the catalytic bed is cooled before being fed to a further conversion step in a second bed. Usually, the cooling medium is a "fresh" reagent having a flow velocity that is high enough to promote a complete mixing with the effluent exiting the catalytic bed in a short amount of time.

Therefore, in light of the considerations above mentioned, it is highly desirable to find a reactor system for industrial applications where adequate mixing conditions can be established inside the reactor independently on the load applied or in other worlds independently on the flow circulating in the system. Additionally, it is highly desirable to find a method for controlling the flow rate of a gas circulating in a reactor system while granting satisfactory mixing conditions inside the reactor even at partial load.

WO 2013/097958 discloses a multi-bed catalytic converter with inter-bed cooling and a related process of conversion of reagents into products. US 5,648,051 discloses an apparatus and method for quenching in hydroprocessing of a hydrocarbon feed stream. WO 99741329 discloses a hydroprocessing reactor and process with liquid quench. US 7,275,565 discloses a multi-conduit multinozzle fluid distributor.

### Summary of the invention

The invention aims to overcome the above drawbacks of the prior art. In particular, the present invention aims to provide a novel reactor system configured to establish satisfactory mixing conditions inside a catalytic converter. The above aim is reached with a reactor system according to claim 1.

Each of the mixing gas feed lines includes at least one flow regulator device so that the amount of mixing gas admitted into the mixing region by each of the feed lines can be independently controlled.

Advantageously, the flow regulator devices allow to independently adjust the flow rate of the mixing gas circulating in each line so that the flow velocity of the mixing gas entering the mixing section is maintained in a target flow velocity range. The target velocity range aims to establish a complete and timely mixing between the inlet feed of said catalytic bed with the mixing gas so that a uniform temperature and composition profile is established in the resulting mixture before reaching the catalyst retained in the catalytic bed of the converter.

Additionally, by maintaining the flow velocity of the mixing gas entering the mixing section in a target range, a uniform distribution of the reactants over the catalyst surface can be established to minimize mass and heat transport limitation.

A further aim of the invention is to provide a method for adjusting the flow rate of a mixing gas circulating in a reactor system wherein the flow rate of the mixing gas fed into the mixing regions of the converter is adjusted by means of the flow regulator devices as a function of the load of the converter.

Advantageously, when the reactor system is subjected to partial load event so when the flow rate of the circulating gas fed to the reactor system decreases (even abruptly), the flow velocity of the makeup gas entering the converter can be maintained in the target velocity range just by acting on the flow regulator devices.

According to the invention, the catalytic converter is a multibed converter including a plurality of catalytic beds and a plurality of mixing regions wherein the number of mixing regions is equal to the number of catalytic beds.

Each mixing region of the catalytic converter is arranged upstream a respective catalytic bed and each mixing region is connected to a respective mixing gas feed line.

According to an embodiment, the mixing region may be confined in a portion of the catalytic bed and arranged upstream of the catalyst so that the mixing between the inlet feed of the catalytic bed and the mixing gas occurs prior to reach the catalyst.

The reactor system comprises mixing gas feed lines configured to feed a mixing gas to the mixing region of the converter. Preferably, said mixing gas feed lines are branched off from a main header so that the mixing gas traversing the main header is split across the mixing gas feed lines. The amount of flow circulating in each mixing line may be controlled by a respective flow regulator device.

According to a preferred embodiment, each mixing region includes a mixing device connected to a respective mixing gas feed line.

Said mixing device is configured to distribute the mixing gas in the mixing region so as to establish satisfactory mixing conditions between the inlet feed of the catalytic bed and the mixing gas. Satisfactory mixing conditions are achieved by maintaining the flow velocity of the mixing gas entering the mixing region to a target value or to a target flow velocity range. The optimal flow velocity range is selected during operation taking into consideration the operating parameter of the converter and the properties of the mixing gas.

According to a preferred embodiment, the mixing device comprises a plurality of flow distributors; each flow distributor is in fluid communication with a respective mixing gas line so that the mixing gas enters the mixing region from separated mixing lines and each distributor is fed individually. The flow rate and therefore the flow velocity of the mixing gas entering the mixing section via each distributor can be regulated or adjusted by the flow regulator devices.

Preferably, the flow distributors are rings or toroids concentrically arranged over each other so to reduce the volume occupied by the mixing device inside the mixing section. This arrangement maximises the room available to the catalytic bed, i.e. the amount of catalyst that can be used.

Preferably, the flow distributors are connected to the mixing device via coaxially arranged tubes to feed the mixing gas to the flow distributors as independent streams. The coaxially arranged tubes allow minimising the volume occupied by the mixing device and reduce the fluid dynamic disturbance that the section of the flow distributors causes to the discharge of the inlet gas into the mixing region.

Preferably the cross-section area and the length of the coaxially arranged tubes are designed to achieve a comparable pressure drop (flowing resistance) when traversed by an equal flow of gas.

Preferably the flow regulator devices are valves that allow a precise regulation of flow (e.g. high quality control valves), preferably having the same relationship between the valve capacity and the plug's displacement (i.e. valve characteristic).

Preferably each valve is provided with its own actuator capable of receiving and translating the signals received from a programmable control unit or a distributed control system into a plug's displacement to close or partially close said valves.

According to a particularly preferred embodiment of the present invention, the total number of flow regulator devices arranged onto the mixing lines is equal to the number of flow distributors of the mixing devices. At least one flow regulator is arranged on each mixing line.

The flow distributor devices may be provided with circular or substantially circular openings having the same cross-sectional area available to the discharge of the mixing gas into the mixing section.

According to a preferred embodiment of the present invention, the reactor system comprises a programmable interface controller or a distributed control system that determines the plug's displacement of the valves to adjust the flow rate (flow velocity) of the mixing gas entering the mixing sections during partial load events.

Preferably, when the mixing gas circulating in the main header decreases, the programmable controller or the distributed control system send a signal to the actuators of the flow control devices so as to close or partially close at least one of the valves located on the mixing gas feed lines. Due to the latter operation, the flow velocity of the mixing gas entering the mixing sections is re-established to the optimal value or in the optimal range.

According to another particularly preferred embodiment of the present invention, the catalytic converter is a multibed converter exploited for the synthesis of ammonia or methanol provided with multiple catalytic beds and multiple mixing sections wherein each mixing section acts as a cooling zone wherein a cold stream is exploited to cool down a reagent mixture or a partially converted reagent mixture.

Preferably, the number of mixing lines connected to each mixing zone is selected as a function of the catalytic converter's operating conditions and depending on the fluid dynamic conditions within the mixing zone. As a general rule, increasing the number of mixing lines connected to a mixing zone results in a more accurate control of the flow velocity resulting in no flow velocity deviation or just in a minimal offset of the flow velocity from the setpoint value.

Additionally, increasing the number of mixing lines communicating within the mixing zone increases the system's ability to accommodate for low reductions in the flow rate of the mixing gas circulating in the reactor system.

Preferably, the most accurate control of the flow velocity of the gas mixture entering the catalytic converter may be required in the mixing section arranged upstream the first catalytic bed, wherein even a slight variation of the operating parameters from the target values may drastically and negatively affect the reaction yield.

According to a particularly preferred embodiment of the present invention, the mixing gas fed to the mixing section may be used as a quenching medium to cool down the inlet gas entering a catalytic bed, being the temperature of said quenching medium lower than the temperature of said inlet gas. According to this embodiment, the flow distributors devices are quench rings.

Preferably, the catalytic converter may comprise a heat exchanger arranged after a bed or enclosed around it so as to maintain a pseudo isothermal temperature profile across the reactive section (i.e. the catalytic bed).

According to an embodiment of the invention, when the catalytic synthesis is carried out via exothermic reactions, the reagent feed line carrying the fresh reagent may be arranged to cross the catalytic converter so to remove the reaction heat by indirect heat transfer from the catalytic bed to the reagent gas so to provide a heated fresh reagent gas at the inlet of the converter.

According to a preferred embodiment of the present invention, the reagent feed line is branched off from the main header to form a network of pipes with the mixing gas feed lines.

Another aspect of the invention is to provide a method for controlling the flow rate of a mixing gas circulating in a reactor system subjected to partial load events. The method of the present invention can be extended to any reactor systems comprising a converter wherein the pressure drop across the mixing section is dominant over the pressure drop measured across the mixing gas feed lines.

The method of the present invention is particularly suited for a reactor system comprising a catalytic converter that operated at high pressure so that the localised pressure drop across each valve is negligible over the absolute pressure of the reactor.

Particularly preferably, an embodiment of the invention is:
a reactor system according to claim 1 with
a plurality of mixing gas feed lines arranged to feed said mixing gas to said mixing region;
wherein each of said mixing gas feed lines includes a valve so that the amount of mixing gas admitted into the mixing region by each of the feed lines can be independently controlled;
wherein each of said mixing gas feed lines are branched off from a main header and preferably the reagent feed line is also branched off from said main header; said mixing region includes a mixing device, said mixing device including distributors arranged in parallel, each of said distributors being fed individually and separately by one of the mixing gas feed lines;
the system further comprises a programmable controller or a distributed control system configured to adjust the aperture or closure of each of said valves as a function of the load of the converter, so that the flow rate of mixing gas fed to each of said distributors is individually controlled.

Particularly preferably, said distributors are ring distributors or toroidal distributors arranged concentrically.

### Figures

Fig. 1 shows an embodiment of the reactor system.
Fig. 2 shows another embodiment of the reactor system.
Fig. 3 shows a further embodiment of the reactor system.
Fig. 4 shows an embodiment of the flow distributors of the mixing device.

### Detailed description of the preferred embodiments

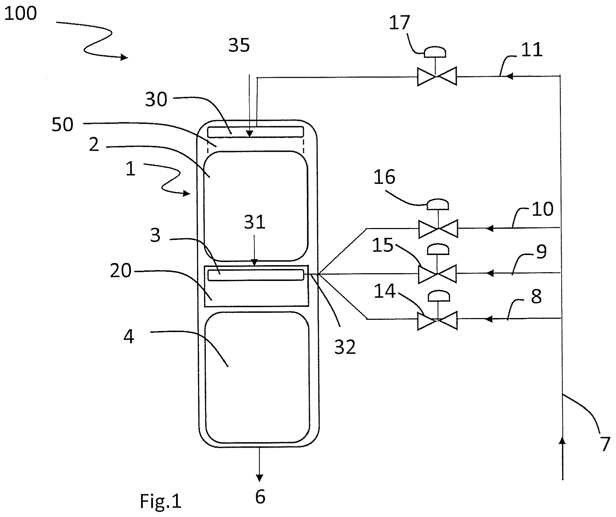

Fig. 1 is a schematic representation of a reactor system 100 of an ammonia synthesis process. The reactor system 100 comprises an ammonia converter 1, a reagent feed line 35 connected to said converter 1 and a plurality of mixing gas feed lines 8, 9, 10, 11.

The reagent feed line 35 and the mixing gas feed lines 8, 9, 10, 11 carry an ammonia make-up gas containing hydrogen and nitrogen (fresh reagent).

The converter 1 is a multibed converter including for example a first catalytic bed 2 and a second catalytic bed 4. In practical case, the converter 1 may include a greater number of beds, for example three or four.

The mixing gas feed lines 8, 9, 10, 11 are branched off from a main header 7. The line 35 may also be branched off from the same header 7. The mixing gas line 11 is in fluid communication with the first catalytic bed 2 via a mixing section 50 of the converter. In this embodiment, for simplicity of representation, the mixing section 50 is shown outside the catalytic bed 2. However, in some configurations, the mixing region 50 can be arranged over the catalyst and contained together with the catalytic bed 2 inside a catalytic basket.

The flow rate in the first mixing gas line 11 can be regulated by a valve 17 whilst the flow rate in the remaining mixing gas lines 8, 9 and 10 can be regulated by respective valves 14, 15, 16.

The mixing gas lines 8, 9, 10 are arranged to feed a portion of the mixing gas from the main header 7 into an inter-bed mixing region 20 downstream of the first catalytic bed 2 and upstream the second catalytic bed 4.

The valves 14, 15, 16 and 17 are operated by a programmable controller or by a distributed control system (not represented in figure) which through its implemented logic regulate the breakdown of the makeup gas carried by line 7 between the mixing gas lines 8, 9, 10, 11.

The inter-bed mixing region 20 is configured to receive a partially converted effluent 31 leaving the first catalytic bed and one or more stream of mixing gas carried by the mixing lines 8, 9 and 10. The mixing gas carried by the mixing lines 8, 9, 10 is fed to the inter-bed coolant section 20 via a flow distributor 3 after traversing a manifold 32.

A plurality of flow sensor devices (e.g. flow meter devices) and pressure sensor devices (e.g. pressure transducers and differential pressure cells) are distributed across the mixing gas feed lines to generate the input signals to the programmable interface control unit or to the distributed control system. Pressure should be preferably detected at least on the mixing line 11, upstream and downstream the valve 17, and flow rate should be measured at least on the main header 7.

The programmable interface control unit or the distributed control system elaborates the input signals received from the sensor devices and provides an output signal to the actuators of the valves 14, 15, 16, 17 to regulate the opening of said valves by adjusting the plug's displacement.

Each valve is provided with its own actuator therefore the flow rate passing through each valve can be adjusted independently accordingly to the output signal provided by the programmable control unit or by the distributed control system.

When the reactor system 100 operates at full capacity, the valves 14, 15, 16, 17 located on the mixing gas line 8, 9, 10, 11 are assumed to be in a mostly opened position in order to assure a proper flow control. When the flow rate carried by the line 7 drops, the flow rate and flow velocity circulating in mixing gas lines 14 to 17 are reduced as well. However, such reduction in not necessarily linear because the resistance to flow (pressure drop) is proportional to the square of the velocity in conventional size pipes.

To restore the flow velocity of the gas entering the mixing section 20 of the converter 1 to the target velocity range two situations are envisaged depending on the flow rate entering in the main header 7.

Specifically, if the reduction in flow rate circulating in line 7 is substantial, at least one of the valves 14, 15, 16 located on the lines 8, 9, 10 closes so that no mixing gas is circulating in the lines where the valves are shut.

Additionally, the valve 17 located on the first mixing line 11 partially closes to create an additional pressure drop on said first line thus compensating the not linear dependence between flow velocity and pressure so that the flow velocity of each portion of mixing gas entering the mixing section 20 is maintained in a target velocity range.

Alternatively, when the reduction in flow rate circulating in the header 7 is only marginal, the valves 14, 15, 16 located on the mixing lines 8, 9, 10 partially close simultaneously so that the mixing gas is distributed in equal portions between said mixing lines 8, 9, 10.

Likewise, the valve 17 located on the first mixing line 11 partially closes to create an additional pressure drop on the first line, and thus maintaining the flow velocity of the gas entering the mixing section 20 to a constant velocity range ideal for establishing good mixing conditions.

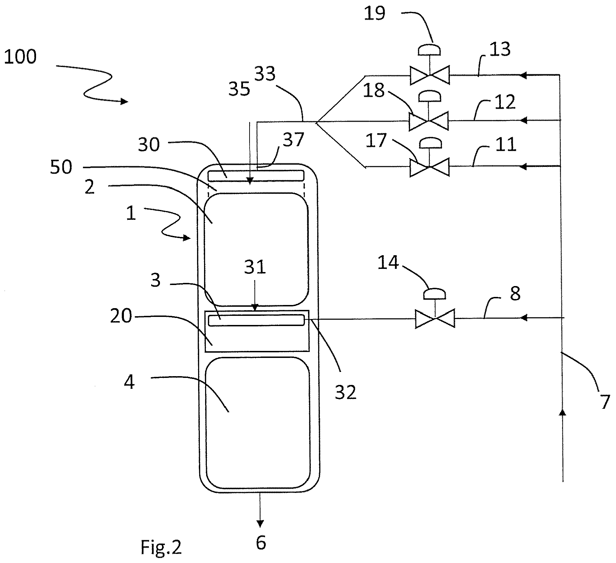

Fig. 2 illustrates another embodiment of the present invention wherein the flow velocity of the mixing gas entering the mixing section 50 arranged above the first catalytic bed 2 is maintained in a target flow velocity range. In this embodiment a good mixing can be established between the reagent gas entering the reagent feed line 35 and the mixing gas carried by the mixing lines 11, 12, 13.

During a partial load event, the valves 17, 18, 19 located on the mixing lines 11, 12, 13 partially close simultaneously or at least one of them close completely depending on the amount of mixing gas entering the main header 7. Instead, the valve 14 located on the mixing line 8 closes only partially, to create an additional pressure drop on the line 8 and compensate for the nonlinear relationship between pressure drop and flow velocity.

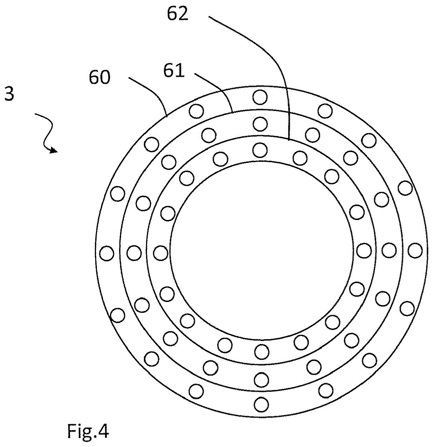

The mixing gas carried by the mixing lines 11, 12, 13 can be distributed in the mixing section 50 of the converter via the mixing device 30 comprising three flow distributors (Fig. 4). The three flow distributors are in fluid communication with the respective mixing lines 11, 12, 13 via a manhole 37 comprising three concentrically arranged tubes, so that each flow distributor is fed separately by one of the lines 11, 12 or 13.

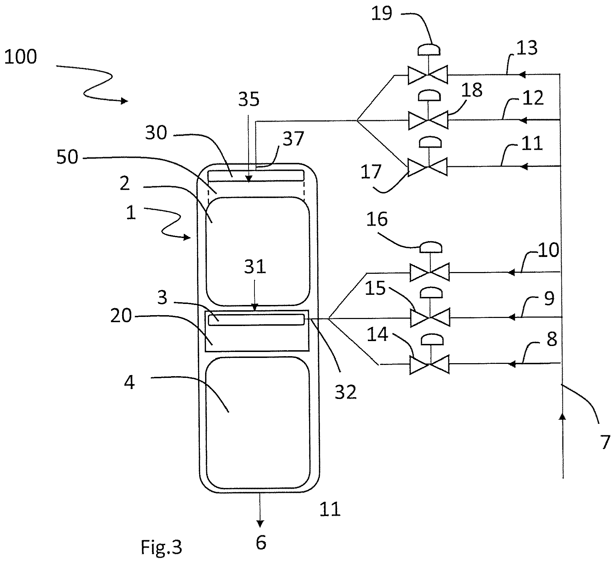

Fig. 3 shows another embodiment of the present invention, wherein the mixing gas feed lines comprise a plurality of lines 8 to 13 communicating with the two mixing sections 20 and 50. Lines 8 to 10 are connected to the mixing section 20 and lines 11 to 13 are connected to the mixing section 50.

In this embodiment, the flow regulator devices 14 to 19 allow maintaining the flow velocity of the mixing gas entering the two mixing sections 20, 50 in a target flow velocity range independently on the flow rate of the mixing gas entering the main header 7.

Fig. 4 shows an embodiment wherein the mixing device 3 comprises toroidal flow distributors 60, 61, 62. Each of said flow distributors is fed individually and separately by one of the feed lines 8, 9 and 10. It is noted that line 32 of Fig. 3 denotes an assembly of three coaxial pipes so that each stream of line 8, line 9 and line 10 can be separately fed to the distributor 60, 61 and 62 respectively. For example line 8 is connected to the distributor 60 via a first pipe of the coaxial assembly 32; line 9 is connected to the distributor 61 via a second pipe of the assembly and line 10 is connected to the distributor 62 via a third pipe of the assembly.

Said flow distributors 60, 61 and 62 are concentrically arranged and provided with circular openings to discharge into the mixing region each portion of the mixing gas circulating in the mixing lines 8, 9, 10.

## Claims

1. A reactor system (100) comprising:
a catalytic converter (1);
a reagent feed line (35) connected to said converter (1);
wherein said converter (1) is a multibed converter comprising a plurality of catalytic beds (2, 4);
wherein said converter further comprises a plurality of mixing regions (20, 50) wherein the number of mixing regions is equal to the number of catalytic beds, each mixing region being arranged upstream of a respective catalytic bed (2, 4) to mix the inlet feed of said catalytic bed with a mixing gas;
a mixing gas feed line (11) or a plurality of mixing gas feed lines (8-10, 11-13) arranged to feed said mixing gas to said mixing regions (20, 50), each mixing region (20, 50) being connected to one or more respective mixing gas feed line(s);
wherein each of said mixing gas feed lines includes at least one flow regulator device (14-16, 17-19), so that the amount of mixing gas admitted into the mixing region by each of the feed lines can be independently controlled.

2. A reactor system (100) according to claim 1, wherein said mixing gas feed lines are branched off from a main header (7) and preferably also the reagent feed line (35) is branched off from said main header (7).

3. A reactor system according to claim 1 or 2, wherein said mixing region (20, 50) includes a mixing device (3, 30) connected to respective mixing gas feed lines (8-10, 11-13).

4. A reactor system (100) according to claim 3, wherein said mixing device (3) comprises a plurality of flow distributors (60, 61, 62), each of said flow distributors being connected in fluid communication with a respective mixing gas feed line (8, 9, 10).

5. A reactor system (100) according to claim 4, wherein said flow distributors (60, 61, 62) are concentrically arranged and preferably have the shape of rings or toroids.

6. A reactor system (100) according to claim 4 or 5, wherein said mixing device (3) is connected to the mixing gas feed lines via a plurality of pipes arranged coaxially, wherein each of the coaxial pipes individually connects one gas feed line with a respective distributor of the mixing device.

7. A reactor system (100) according to any of the previous claims, wherein said flow regulator device(s) (14 to 19) is/are valves.

8. A reactor system (100) according to any of the previous claims, further comprising a programmable controller or a distributed control system configured to adjust the flow rate through the flow regulator device(s) as a function of the load of the converter (1).

9. A reactor system according to claims 7 and 8, wherein the control system is configured to adjust the opening degree of the valves.

10. A reactor system (100) according to any of the previous claims, wherein the mixing gas feed lines (8 to 13) are quench lines configured to carry a mixing gas having a temperature lower than the temperature of the feed stream of the catalytic bed, so that the mixing gas can be used as a quench gas.

11. A reactor system (100) according to any of the previous claims, wherein the catalytic converter (1) is adapted for the synthesis of ammonia or the catalytic converter (1) is adapted for the synthesis of methanol.

12. A reactor system (100) according to any of the previous claims, further comprising a heat exchanger arranged downstream a catalytic bed (2) to remove heat from the effluent of said catalytic bed.

13. Method to adjust the flow rate circulating in a reactor system (100) according to any of claims 1 to 12, wherein the flow rate of the mixing gas fed into the mixing regions of the converter (1) is adjusted by the flow regulator device(s) (14 to 19) as a function of the load of the converter (1).

## Patentansprüche

1. Ein Reaktorsystem (100), umfassend:
einen katalytischen Konverter (1);
eine Reagenz-Zufuhrleitung (35), die mit dem Konverter (1) verbunden ist; wobei der Konverter (1) ein Mehrbettkonverter ist, der eine Vielzahl von katalytischen Betten (2, 4) umfasst;
wobei der Konverter ferner eine Vielzahl von Mischbereichen (20, 50) umfasst, wobei die Anzahl der Mischbereiche gleich der Anzahl der katalytischen Betten ist, wobei jeder Mischbereich stromaufwärts eines jeweiligen katalytischen Bettes (2, 4) angeordnet ist, um die Einlasszufuhr des katalytischen Bettes mit einem Mischgas zu mischen;
eine Mischgas-Zufuhrleitung (11) oder eine Mehrzahl von Mischgas-Zufuhrleitungen (8-10, 11-13), die so angeordnet sind, dass sie das Mischgas den Mischbereichen (20, 50) zuführen, wobei jeder Mischbereich (20, 50) mit einer oder mehreren jeweiligen Mischgas-Zufuhrleitung(en) verbunden ist;
wobei jede der Mischgas-Zufuhrleitungen mindestens eine Durchflussregelvorrichtung (14-16, 17-19) umfasst, so dass die Menge an Mischgas, die von jeder der Zufuhrleitungen in den Mischbereich eingeleitet wird, unabhängig gesteuert werden kann.

2. Reaktorsystem (100) nach Anspruch 1, wobei die Mischgas-Zufuhrleitungen von einem Hauptverteiler (7) abzweigen und vorzugsweise auch die Reagenz-Zufuhrleitung (35) von dem Hauptverteiler (7) abzweigt.

3. Reaktorsystem nach Anspruch 1 oder 2, wobei der Mischbereich (20, 50) eine Mischvorrichtung (3, 30) umfasst, die mit entsprechenden Mischgas-Zufuhrleitungen (8-10, 11-13) verbunden ist.

4. Reaktorsystem (100) nach Anspruch 3, wobei die Mischvorrichtung (3) eine Vielzahl von Strömungsverteilern (60, 61, 62) umfasst, wobei jeder der Strömungsverteiler in Fluidverbindung mit einer jeweiligen Mischgas-Zufuhrleitung (8, 9, 10) verbunden ist.

5. Reaktorsystem (100) nach Anspruch 4, wobei die Strömungsverteiler (60, 61, 62) konzentrisch angeordnet sind und vorzugsweise die Form von Ringen oder Tori haben.

6. Reaktorsystem (100) nach Anspruch 4 oder 5, wobei die Mischvorrichtung (3) mit den Mischgas-Zufuhrleitungen über eine Mehrzahl von koaxial angeordneten Rohren verbunden ist, wobei jedes der koaxialen Rohre individuell eine Gaszufuhrleitung mit einem jeweiligen Verteiler der Mischvorrichtung verbindet.

7. Ein Reaktorsystem (100) nach einem der vorherigen Ansprüche, wobei die Durchflussregelvorrichtung(en) (14 bis 19) Ventile sind.

8. Ein Reaktorsystem (100) nach einem der vorherigen Ansprüche, das ferner eine programmierbare Steuerung oder ein verteiltes Steuersystem umfasst, das so konfiguriert ist, dass es die Durchflussrate durch die Durchflussregelvorrichtung(en) als Funktion der Last des Konverters (1) anpasst.

9. Ein Reaktorsystem nach den Ansprüchen 7 und 8, wobei das Steuersystem so konfiguriert ist, dass es den Öffnungsgrad der Ventile anpasst.

10. Reaktorsystem (100) nach einem der vorhergehenden Ansprüche, wobei die Mischgas-Zufuhrleitungen (8 bis 13) Quenchleitungen sind, die so konfiguriert sind, dass sie ein Mischgas mit einer Temperatur führen, die niedriger ist als die Temperatur des Zufuhrstroms des katalytischen Bettes, so dass das Mischgas als Quenchgas verwendet werden kann.

11. Ein Reaktorsystem (100) nach einem der vorherigen Ansprüche, wobei der katalytische Konverter (1) für die Synthese von Ammoniak oder der katalytische Konverter (1) für die Synthese von Methanol angepasst ist.

12. Ein Reaktorsystem (100) gemäß einem der vorherigen Ansprüche, das ferner einen Wärmetauscher umfasst, der stromabwärts eines katalytischen Bettes (2) angeordnet ist, um dem Abfluss des katalytischen Bettes Wärme zu entziehen.

13. Verfahren zum Einstellen der Durchflussrate, die in einem Reaktorsystem (100) zirkuliert, gemäß einem der Ansprüche 1 bis 12, wobei die Durchflussrate des Mischgases, das in die Mischbereiche des Konverters (1) eingespeist wird, durch die Durchflussregelvorrichtung(en) (14 bis 19) als eine Funktion der Last des Konverters (1) eingestellt wird.

## Revendications

1. Système de réacteur (100), comprenant :
un convertisseur catalytique (1) ;
une conduite d'alimentation en réactif (35) raccordée audit convertisseur (1) ;
dans lequel ledit convertisseur (1) est un convertisseur multi-lits comprenant une pluralité de lits catalytiques (2, 4) ;
dans lequel ledit convertisseur comprend en outre une pluralité de régions de mélange (20, 50), dans lequel le nombre de régions de mélange est égal au nombre de lits catalytiques, chaque région de mélange étant agencée en amont d'un lit catalytique respectif (2, 4) pour mélanger l'alimentation d'entrée dudit lit catalytique à un gaz de mélange ;
une conduite d'alimentation en gaz de mélange (11) ou une pluralité de conduites d'alimentation en gaz de mélange (8-10, 11-13) agencée(s) pour alimenter ledit gaz de mélange vers lesdites régions de mélange (20, 50), chaque région de mélange (20, 50) étant raccordée à une ou plusieurs conduite(s) d'alimentation en gaz de mélange respective(s) ;
dans lequel chacune desdites conduites d'alimentation en gaz de mélange comprend au moins un dispositif régulateur de débit (14-16, 17-19), de sorte que la quantité de gaz de mélange admise dans la région de mélange par chacune des conduites d'alimentation puisse être contrôlée indépendamment.

2. Système de réacteur (100) selon la revendication 1, dans lequel lesdites conduites d'alimentation en gaz de mélange sont dérivées à partir d'un collecteur principal (7) et de préférence également la conduite d'alimentation en réactif (35) est dérivée à partir dudit collecteur principal (7).

3. Système de réacteur selon la revendication 1 ou 2, dans lequel ladite région de mélange (20, 50) comprend un dispositif de mélange (3, 30) raccordé à des conduites d'alimentation en gaz de mélange respectives (8-10, 11-13).

4. Système de réacteur (100) selon la revendication 3, dans lequel ledit dispositif de mélange (3) comprend une pluralité de répartiteurs de débit (60, 61, 62), chacun desdits répartiteurs de débit étant raccordé en communication fluidique avec une conduite d'alimentation en gaz de mélange respective (8, 9, 10).

5. Système de réacteur (100) selon la revendication 4, dans lequel lesdits répartiteurs de débit (60, 61, 62) sont agencés concentriquement et ont de préférence la forme de bagues ou de tores.

6. Système de réacteur (100) selon la revendication 4 ou 5, dans lequel ledit dispositif de mélange (3) est raccordé aux conduites d'alimentation en gaz de mélange par l'intermédiaire d'une pluralité de tuyaux agencés de manière coaxiale, dans lequel chacun des tuyaux coaxiaux raccorde individuellement une conduite d'alimentation en gaz à un répartiteur respectif du dispositif de mélange.

7. Système de réacteur (100) selon l'une quelconque des revendications précédentes, dans lequel ledit/lesdits dispositif(s) régulateur(s) de débit (14 à 19) est/sont des soupapes.

8. Système de réacteur (100) selon l'une quelconque des revendications précédentes, comprenant en outre un contrôleur programmable ou un système de contrôle réparti configuré pour ajuster le débit à travers le(s) dispositif(s) régulateur(s) de débit en fonction de la charge du convertisseur (1).

9. Système de réacteur selon les revendications 7 et 8, dans lequel le système de contrôle est configuré pour ajuster le degré d'ouverture des soupapes.

10. Système de réacteur (100) selon l'une quelconque des revendications précédentes, dans lequel les conduites d'alimentation en gaz de mélange (8 à 13) sont des conduites de refroidissement configurées pour transporter un gaz de mélange ayant une température inférieure à la température du flux d'alimentation du lit catalytique, de sorte que le gaz de mélange puisse être utilisé en tant qu'un gaz de refroidissement.

11. Système de réacteur (100) selon l'une quelconque des revendications précédentes, dans lequel le convertisseur catalytique (1) est adapté à la synthèse de l'ammoniac ou le convertisseur catalytique (1) est adapté à la synthèse du méthanol.

12. Système de réacteur (100) selon l'une quelconque des revendications précédentes, comprenant en outre un échangeur de chaleur agencé en aval d'un lit catalytique (2) pour évacuer de la chaleur à partir de l'effluent dudit lit catalytique.

13. Procédé pour ajuster le débit circulant dans un système de réacteur (100) selon l'une quelconque des revendications 1 à 12, dans lequel le débit du gaz de mélange alimenté dans les régions de mélange du convertisseur (1) est ajusté par le(s) dispositif(s) régulateur (s) de débit (14 à 19) en fonction de la charge du convertisseur (1).
